# EUROPEAN PATENT SPECIFICATION

(11) **EP 0 714 418 B1**
(45) Date of publication and mention of the grant of the patent: **25.10.2000**
(21) Application number: 94925861.0
(22) Date of filing: 11.08.1994
(51) Int. Cl.: C08G 63/06, C08G 65/38, A01N 43/38, A01N 47/28, A61K 7/42, A61K 7/021, A61K 31/175, C12P 1/00, C07D 209/04, G01N 33/00

(54) **COSMETIC MELANINS**
KOSMETISCHE MELANINE
MELANINES COSMETIQUES

(30) Priority: 19.08.1993 US 109286
(43) Date of publication of application: 05.06.1996
(73) Proprietor: YALE UNIVERSITY, New Haven CT 06520 (US)
(72) Inventor: PAWELEK, John, M., Hamden, CT 06517 (US)
(74) Representative: Adams, Nicola
(86) International application number: US9409153
(87) International publication number: WO9505412

(56) References cited:
- EP-A- 0 356 119
- EP-A- 0 386 680
- US-A- 4 515 773
- US-A- 4 656 029
- US-A- 4 826 677
- US-A- 4 855 144
- US-A- 5 227 459
- US-A- 5 252 628
- US-A- 5 256 403
- JOURNAL OF AMERICAN CHEMICAL SOCIETY, Volume 106, issued 1984, R.M.B. DEIBEL et al.: "Biosynthetic and structural Studies on Pheomelanin.2", pages 5884-5888, especially page 5885.
- J. CHEM. SOC., FARADAY, TRANS. 2, Volume 82, issued 1986, J. BIELEC et al., "Photochemical Studies on Porphyrin-Melanin Interactions", pages 1469-1474, especially abstract.
- J. CHEM. SOC., CHEM. COMMUN., Volume 17, issued 1984, M.R. CHEDEKEL et al.: "Early Steps in the Free Radical Polymerisation of 3,4-Dihydroxyphenylalanine (Dopa) into Melanid", pages 1170-1172, especially abstract.
- G. PROTA, "Melanins and Melanogenesis", published 1984 by ACADEMIC PRESS INC. (SAN DIEGO), pages 1-13 and 63-87, especially page 11.

## Description

### Field of the Invention

The present invention concerns the synthesis of soluble forms of melanin and their compositions, and methods using such compositions to provide substantive, natural-appearing colors to mammalian skin and to protect skin against ultraviolet radiation.

### Background Information

It is well established that the incidence of melanoma and other cancers of the skin is on the rise, and that solar radiation, particularly that in the ultraviolet (UV) range, is a major causative factor (Pawelek et al., Molecular cascades in UV-induced melanogenesis: A central role for melanotropins?, Pigment Cell Research, 5:348-356, 1992). In this regard, individuals with high skin melanin content are less likely to suffer from skin cancers and other solar-induced damage (wrinkling, solar lentigines, "aging"), than those with low skin melanin content.

Public awareness of these facts has led to an increased usage of commercial sunscreens that protect the skin from the penetration of UV. However, many individuals who use sunscreens also desire to have tan-appearing skin, and the active ingredients of most of these sunscreens do not impart a tan to the skin. On the other hand, there are a variety of agents commercially available which provide a tan color to the skin, but few, if any of these, provide significant protection from UV. For example, dihydroxyacetone (DHA) is by far the most prevalent active ingredient of all the "tanning" products sold. While DHA can impart a melanin-like color to the skin, it exhibits no protection from UV. Nonetheless, the commercial market for DHA is vast, attesting to a desire among a large number of people to have tan skin.

Wolfram et al. (US Patent 4,968,497) disclose tanning by applying a composition comprising melanin precursors or melanin precursor-like materials to the skin. They do not teach or claim that applying melanins, either natural or synthetic, to the skin will mimic skin tanning. Rather they teach that such melanins achieve "only superficial external tanning results which is readily removed by rinsing with water or rubbing with a towel".

Herlihy (US Patent 4,515,773) discloses a skin tanning composition containing a melanin precursor and a tyrosinase enzyme in a cosmetic base. He does not, however, teach or claim the application of melanins, either natural or synthetic to the skin.

Gaskin (US Patent 4,806,344) discloses a sun protectant composition comprising "melanin" as one of the ingredients. Gaskin further discloses that the melanin is "synthesized from tyrosinase and DOPA". She does not teach or suggest that such melanin is synthesized non-enzymatically or that it is comprised of precursors which confer either negative or positive charges on the polymer, rendering it soluble in aqueous solutions.

Ahene and Chedekel (US Patent 5,188,844) disclose color modification of pre-formed melanins as a method for producing melanins of various hues. They subject preformed melanins, either synthetic or natural, to a variety of chemical procedures that result in color modifications of the melanins. They do not teach or claim that synthetic melanins of various hues can be produced by polymerization of one or more appropriate precursors, yielding melanins of predictable hues and molecular weights that require no further chemical modifications.

Mammalian melanins have proven difficult to study because their solubilization often requires destructive treatments such as boiling in strong alkali, or the use of strong oxidants such as hydrogen peroxide. In this regard Prota (Prota, Giuseppe, 1992, Melanins and Melanogenesis, Academic Press, Inc. Harcourt Brace Jovanovich, Publishers, San Diego 290 pp.--specifically Chapter 1: II the Intractable Nature of Melanins, pp. 3-4) writes:
"Several obstacles account for the slow progress that marks melanin research. First, natural melanins are highly insoluble materials of presumably high molecular weight and are therefore difficult to separate from the other cellular components of the organism in which they occur. Even when a melanin has been isolated, it is difficult to know whether or not it is pure, if indeed the term "pure" can rightly be applied to any melanin".

Prota (Chapter 4: IV pp 73-74) further teaches that studies on the protection of synthetic melanins have been restricted to a small number of monomeric precursors and have usually, but not always, employed enzymatic polymerization techniques. He writes:
"A laboratory procedure which has been widely used to obtain eumelaninlike [sic] pigments involves oxidation of L-tyrosine or L-dopa at neutral pH in the presence of tyrosinase, usually the commercially available mushroom enzyme"... "Various methods of synthesis have been used, including 1) auto oxidation in the presence or in the absence of metal ions; 2) enzymic oxidation with tyrosinase or peroxidase; and 3) anodic oxidation in both acidic and neutral media". (pp 73-74)

In contrast, the current invention teaches that high molecular weight molecules displaying physical/chemical characteristics of melanins can be prepared free of enzymes and other proteins by at least six specific non-enzymatic procedures, with each procedure yielding reproducibly different melanins of predictable colors, spectra, and molecular weights. The current invention also teaches that the starting precursors for soluble melanin synthesis belong to a class of compounds defined as having aromatic rings with ionizeable side groups. Furthermore, during the polymerization of such compounds, additional molecular entities are incorporated into the melanin polymeric structure, thereby improving properties of the melanin such as substantivity to skin and hair, or color/hue characteristics. Such advantageous incorporation of molecular entities can be achieved by the non-enzymatic procedures, with each procedure yielding reproducibly different melanins. The current invention teaches that such melanins can be readily synthesized in industrial-sized quantities.

Pawelek et al., US Patent 5,218,079, and later Orlow, Osber and Pawelek, 1992, Pigment Cell Research 5: 113-121, Synthesis and Characterization of Melanins from Dihydroxyindole-2-Carboxylic Acid and Dihydroxyindole, described conditions wherein melanins can be synthesized non-enzymatically using DHICA and/or DHI as the monomeric precursors, and that the resulting synthetic melanins are comprised of 100-1000 monomeric subunits of the precursors. It was further shown that the presence of negatively charged (non-protonated) carboxylic acid residues on the DHICA monomers enhanced the solubility of DHICA-melanins in aqueous solutions above pH 5.

Pawelek et al. (US Patent Application Serial No. 016,330, filed February 11, 1993, now pending) described conditions wherein melanins can be synthesized non-enzymatically using DHICA and/or a phenethylamine such as 3-amino-tyrosine and that the presence of positively charged (protonated) amino residues on the 3-amino-tyrosine monomers also enhanced the solubility of the resulting melanin polymers in aqueous solutions over a broad pH range. Polymerization of such melanins was carried out above pH 7 in the presence of oxygen, and was enhanced by the additions of oxidative agents such as hydrogen peroxide and also by the salts of metals such as Cu²⁺. The presence of 3-amino-tyrosine monomers in synthetic melanins imparted a red golden hue to the melanin. Pawelek et al. taught that additional synthetic soluble melanins can be created using suitable analogs to DHICA or 3-amino-tyrosine wherein ionizing side groups, such as phosphate or sulfate groups, are present on the precursor molecules. Pawelek et al. further taught that such soluble synthetic melanins could be utilized for a number of different purposes, including uses as cosmetics to impart natural-appearing colors to mammalian skin and hair, as well as to provide protection from ultraviolet light. Pawelek et al. further taught that one method for increasing the adherence (substantivity) of such melanins to mammalian skin is to mix said melanins with dihydroxyacetone, which apparently acts as a crosslinking agent to covalently link the melanins to various components of skin and hair, e.g. proteins of the skin.

While the resulting melanins described in the above-identified disclosures have many advantages in their performance, they still have certain limitations in their range of colors, as well as in their substantivity to skin and hair.

The present invention provides a synthetic melanin polymer comprising units derived from at least one monomer selected from 3-aminotyrosine, dihydroxybenzoic acid, 3-amino-4-hydroxybenzoic acid, tyrosine, dihydroxyphenylalanine, dihydroxynapththalene sulfonic acid, 3-nitrotyrosine, 3-dimethylamino phenol and p-aminobenzoic acid, and from at least one co-monomer selected from cholesterol, dihydroxycarbazole, aloin, emodin, linoleic acid and linolenic acid, the co-monomer(s) having been co-polymerized with the monomer(s), the polymer being substantive to human skin and hair and being soluble in an aqueous cosmetic buffer at physiological pH and temperature.

The synthetic melanin polymers of the present invention, referred to herein as melanins, are synthetic melanins that impart true melanin colors to the skin and are suitable for commercialization as "cosmetic melanins". While such melanins have a number of uses and advantages, perhaps the most significant of these is to impart a tan to the skin while at the same time provide protection from UV radiation.

This present invention concerns synthetic cosmetic melanins that satisfy the criteria listed below.
1) Natural-appearing colors
2) Adaptive to different skin/hair colorations
3) Natural and/or organic composition
4) High molecular polymers, free of monomers and reagents
5) Ultraviolet radiation absorbent
6) Soluble in cosmetic buffers (e.g. triethanolamine, sodium phosphate) at physiologic pH and temperature
7) Ease of application
8) Substantive to skin (water/soap resistant)
9) Non-spreading from skin to clothing/towels
10) Non-mutagenic
11) Non-irritant, non-allergenic
12) Feasible industrial-scale production
13) Environmentally safe

Although some of these criteria have been satisfied by previous additives, no simple component has satisfied all of these criteria, which together constitute a complete definition of "cosmetic melanin".

The present invention further provides a method of obtaining a synthetic melanin polymer, which comprises mixing at least one monomeric material selected from 3-aminotyrosine, dihydroxybenzoic acid, 3-amino-4-hydroxybenzoic acid, tyrosine, dihydroxyphenylalanine, dihydroxynaphthalene sulfonic acid, 3-nitrotyrosine, 3-dimethylamino phenol and p-aminobenzoic acid with at least one co-monomer selected from cholesterol, dihydroxycarbazole, aloin, emodin, linoleic acid and linolenic acid and polymerizing the mixture in aqueous solution by oxidation in the presence of air or oxygen:
(a) in the presence of a weak base; or
(b) in the presence of a weak base and a salt of Cu⁺⁺ or Fe⁺⁺; or
(c) in the presence of a weak base, a salt of Cu⁺⁺ or Fe⁺⁺ and an oxidant; or
(d) in the presence of a strong base; or
(e) in the presence of a strong base and a salt of Cu⁺⁺ or Fe⁺⁺; or
(f) in the presence of a strong base, a salt of Cu⁺⁺ or Fe⁺⁺ and an oxidant.

The method may comprise a further step in which an alcohol, preferably ethanol, is added to the resulting solution of formed polymer thereby to precipitate the polymer from said solution.

This series of distinctly different methods for the oxidative polymerization of melanin precursors yield reproducibly different melanins, even when the same precursors are used as starting material. Although it is well known that melanins can be synthesized through oxidative polymerization of appropriate precursors such as, for example, dihydroxyphenylalanine, it was not previously known until this invention that different procedures of oxidative polymerization could yield melanins with reproducibly different physical characteristics such as molecular weight and color. Nor was it known that a wide variety of compounds from different chemical classes can be polymerized or copolymerized through such procedures and that said procedures can thus be used together to screen for new melanins, polymerized from different precursors or combinations thereof. A key factor in the development of the above-mentioned cosmetic melanins was availability of techniques for the synthesis of water-soluble melanins described in Patent No. 5,218,079.

Also provided by the present invention is a method of cosmetically producing a naturally appearing tan on mammalian skin comprising applying to the skin a tan-producing effective amount of a synthetic melanin polymer comprising units derived from at least one monomer selected from 3-aminotyrosine, dihydroxybenzoic acid, 3-amino-4-hydroxybenzoic acid, tyrosine, dihydroxyphenylalanine, dihydroxynapththalene sulfonic acid, 3-nitrotyrosine, 3-dimethylamino phenol and p-aminobenzoic acid, and from at least one co-monomer selected from cholesterol, dihydroxycarbazole, aloin, emodin, linoleic acid and linolenic acid, the co-monomer(s) having been co-polymerized with the monomer(s), the polymer being substantive to human skin and hair and being soluble in an aqueous cosmetic buffer at physiological pH and temperature.

It is accordingly an object of the present invention to provide additional synthetic forms of melanins that are soluble at physiologic pH and temperatures.

It is another object of this invention to provide melanin polymers whose monomeric structures are aromatic compounds with ionizable side groups.

It is another object of the present invention to provide melanins and methods for making such melanins having a wide variety of natural-appearing colors.

It is another object of the present invention to provide melanins with increased adherence to mammalian skin.

It is another object of the present invention to provide methods for the co-polymerization of synthetic melanin monomers with agents that enhance certain properties of the resultant melanin polymers, examples being enhanced adherence to skin, enhanced protection from ultraviolet light, and modification of color.

It is another object of the present invention to provide methods for producing such melanins in industrial-scale quantities.

It is another object of this invention to reduce the incidence of solar damage of the skin (aging, solar lentigines, wrinkling, cancer) in the human population through the commercial distribution of said cosmetic melanins.

The above objects and other objects, aims and advantages are satisfied by the present invention. The synthetic polymers of the present invention exhibit physico-chemical properties suitable for cosmetic applications including natural-appearing colors, solubility in aqueous buffers at physiologic pH and temperatures, substantivity to human skin and hair, and protection from ultraviolet radiation. Synthetic melanin polymers according to the invention have monomeric structures which are composed of aromatic rings that possess ionizeable side groups. The present invention also relates to six specific non-enzymatic methods for co-polymerization of monomers and co-monomers into synthetic melanins. Said methods can also be used to screen monomeric structures for their incorporation into synthetic melanin polymers. They may be used as methods for producing such melanins in industrial scale quantities. The present invention also relates to methods, i.e. cosmetic application, that reduce the incidence of solar damage to the skin in the human population.

### Detailed Description of the Invention

The polymers of the invention are produced through any one of six separate procedures. Said procedures involve oxidation in the presence of air (or oxygen) under the following conditions:
a) In the presence of a weak base such as ammonium hydroxide or triethanolamine;
b) In the presence of a weak base such as ammonium hydroxide or triethanolamine and a salt of Cu⁺⁺ or Fe⁺⁺;
c) In the presence of a weak base such as ammonium hydroxide or triethanolamine, a salt of Cu⁺⁺ or Fe⁺⁺, and an oxidant such as hydrogen peroxide or ammonium persulfate;
d) In the presence of a strong base such as sodium hydroxide or potassium hydroxide;
e) In the presence of a strong base such as sodium hydroxide or potassium hydroxide and a salt of Cu⁺⁺ or Fe⁺⁺;
f) In the presence of a strong base such as sodium hydroxide or potassium hydroxide, a salt of Cu⁺⁺ or Fe⁺⁺, and an oxidant such as hydrogen peroxide or ammonium persulfate.

Then a determination is made to see if the product of any of a) to f) is a polymer and is substantive to human skin and hair (These procedures a) to f) are referred to below as procedures 1 to 6.)

Generally, the precursors most amenable to polymerization through these procedures have hydroxylated aromatic rings with ionizeable side groups. As discussed more fully hereinbelow, with reference to the drawings one such precursor (monomer), p-aminobenzoic acid (PABA) is not hydroylated, yet it can nonetheless be polymerized into melanin by two of the six methods. The finding serves to demonstrate that the cosmetic melanins provide protection from UV, since PABA is widely used as a sunscreen, and PABA-melanin absorbs over a broader range of the UV spectrum than does PABA itself (see below for further discussion of this point).

Other suitable precursors (monomers) are tyrosine, 3-amino-tyrosine, 3-nitro-tyrosine, dihydroxyphenylalanine, dihydroxybenzoic acid, 3-amino-4-hydroxybenzoic acid, 3-dimethylamino phenol and dihydroxynaphthalene sulphonic acid.

Molecules that enhance penetration and substantivity of synthetic melanins to skin are copolymerized along with the precursors, yielding melanins that are uniquely applicable to cosmetic usage. Such molecules are linoleic acid, and linolenic acid (fatty acids), cholesterol (a steroid), dihydroxycarbazole (a carbazole alkaloid) and "aloin" and "emodin" which are known to be ingredients of Aloe Vera and related plants. Examples of additional enhancing agents that can be included in the polymerization reactions with the comonomers include aromatic glycosides such as those found in extracts of the Aloe Vera plant and related plants.

Aloin and emodin function as both melanin precursors and as melanin enhancers. If polymerized alone they yield melanin-like compounds with gold and red colors. When, as in the present invention, they are copolymerized with other precursors, e.g. dihydroxyphenylalanine (dopa), they enhance the substantivity of the dopa-melanin polymer to the skin.

### Brief Description of the Drawings The invention will be further described with reference to the accompanying drawings wherein:

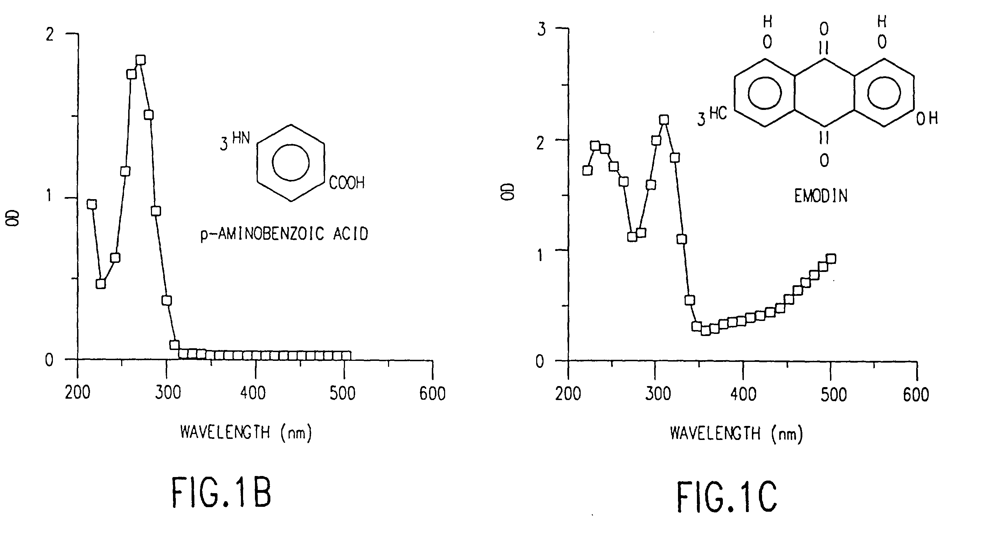

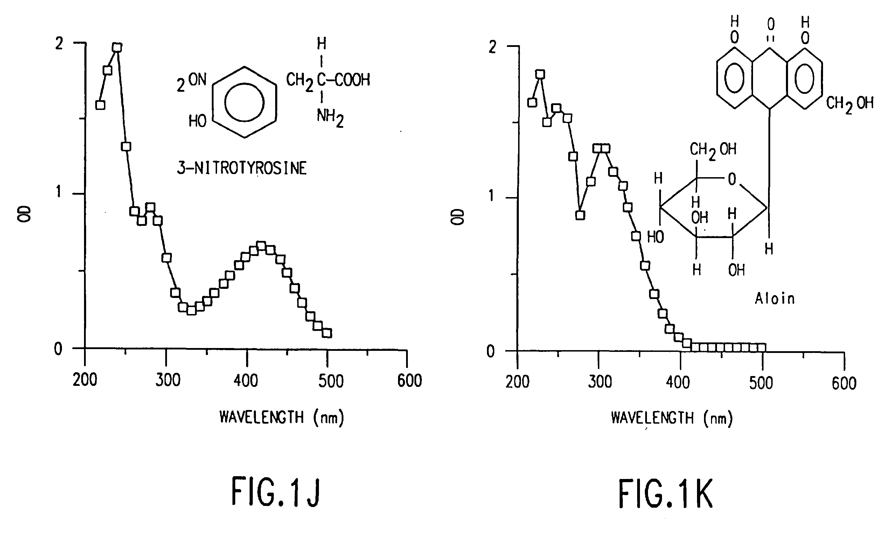

- Pig. 1 (A) to (K):: Shows the chemical structure and spectra of representative precursors and two co-monomers (melanin-enhancing agents, see Figs. 1 (C) and (K)) used in the production of chemical melanins;
- Fig. 2:: Shows the spectral characteristics of p-aminobenzoic acid and a synthetic, melanin-like polymer of p-aminobenzoic acid;
- Fig. 3:: Shows the chemical structures of "melanin enhancing" agents (comonomers) used in the production of the polymers of the present invention.
- Fig. 4:: Shows the spectral characteristics of cosmetic melanins of various colors; and
- Fig. 5:: Shows plots of molecular weights vs. absorbance (450nm) of melanin polymers.

### Detailed Description of the Drawings

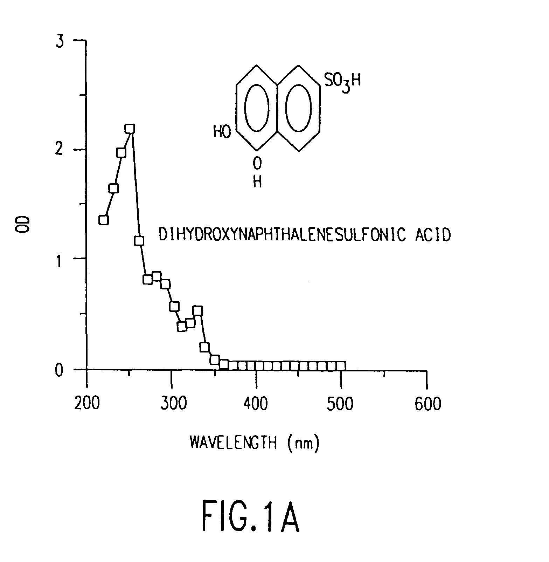

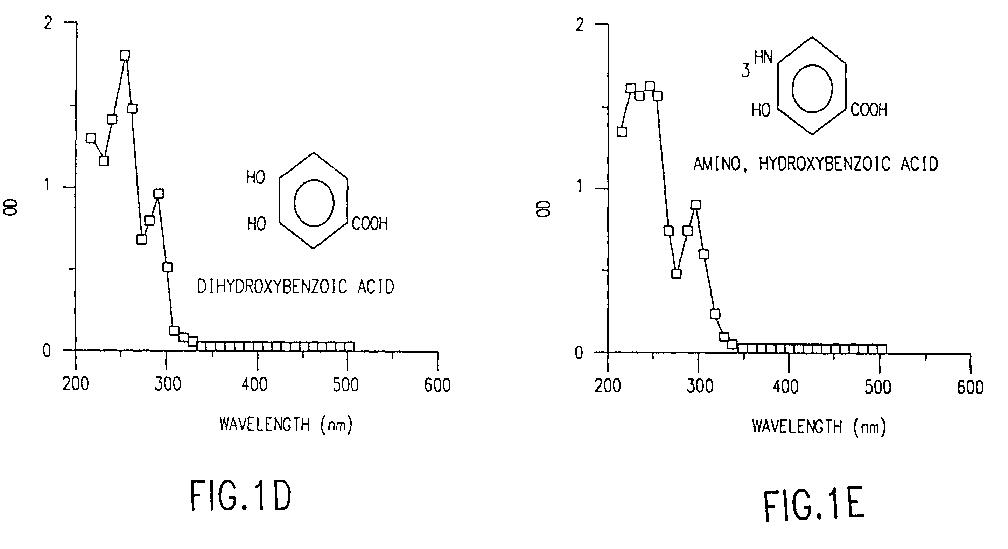

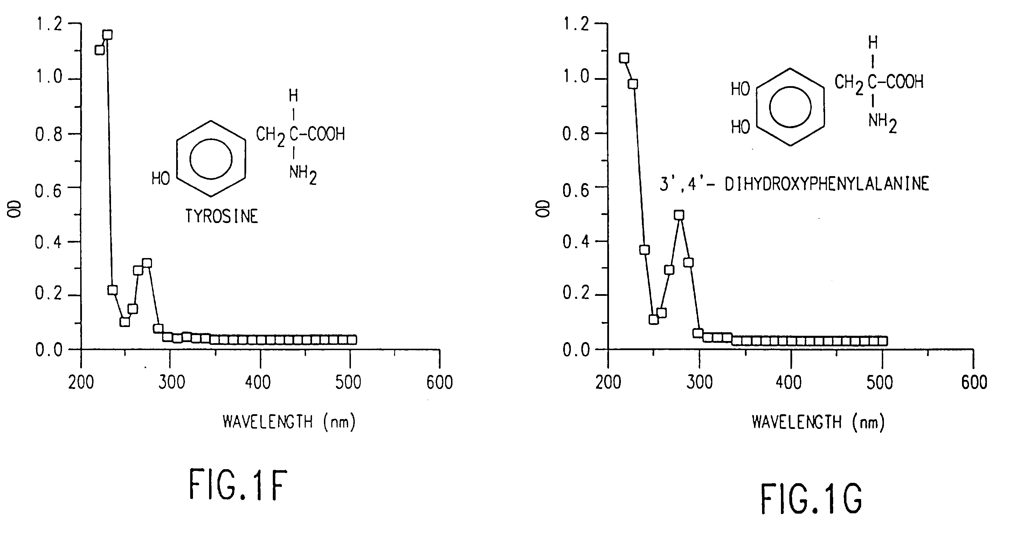

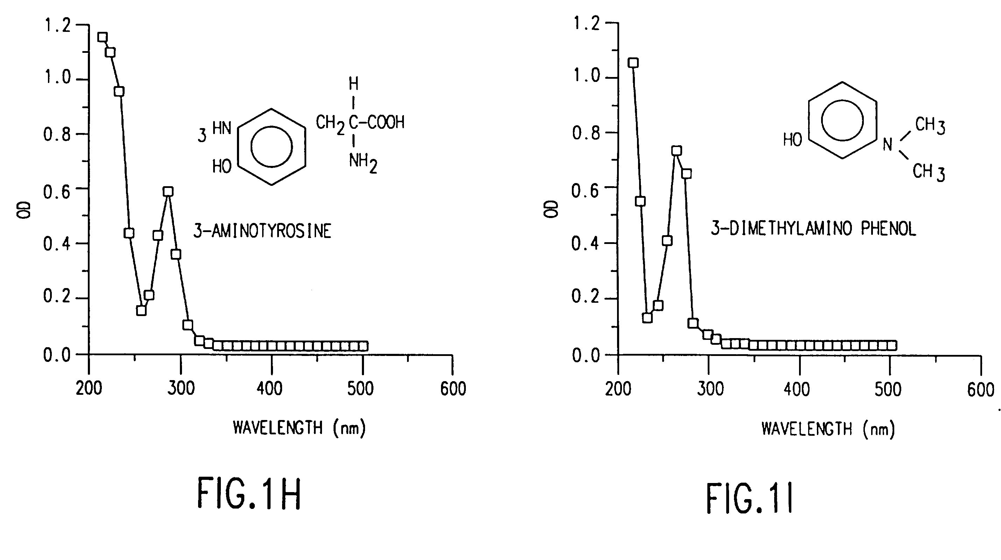

- Fig. 1:: Chemical structures and spectrophotometric characteristics of representative precursors used in the production of cosmetic melanins. The precursors share the common characteristics of possessing at least one aromatic ring and at least one ionizeable side group. Except for P-aminobenzoic acid, the precursors also contain hydroxyl groups. Each of the precursors absorbs light in the ultraviolet spectra.

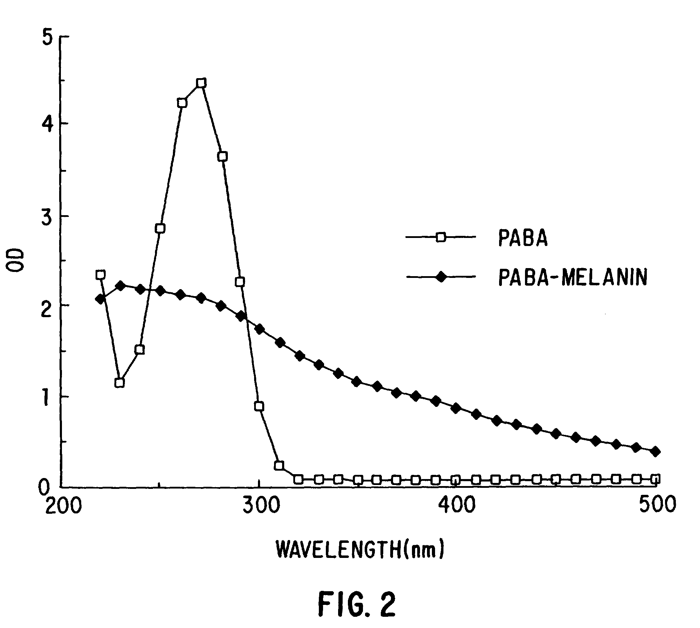

- Fig. 2:: Spectral characteristics of p-aminobenzoic acid and a synthetic melanin-like polymer of p-aminobenzoic acid. Although the melanin-like polymer of p-aminobenzoic acid does not fall within the present claims, the matters illustrated by the polymer are relevant to the present invention. Even though PABA does not contain a hydroxylated moiety, it could be polymerized into a high molecular weight melanin-like polymer by procedures #3 (weak base, salt of a metal ion, oxidant) and #5 (strong base, salt of metal ion) listed above. The dried powders of PABA and PABA-polymers were separately dissolved in 0.1M sodium phosphate, pH 7.4 at a concentration of 50µg/ml, and absorbance was determined in a spectrophotometer over the range of wavelengths shown. The melanin-like PABA polymer showed a much broader absorbance range than did PABA itself, demonstrating that the polymer is a superior absorbent of light when compared to the precursor.

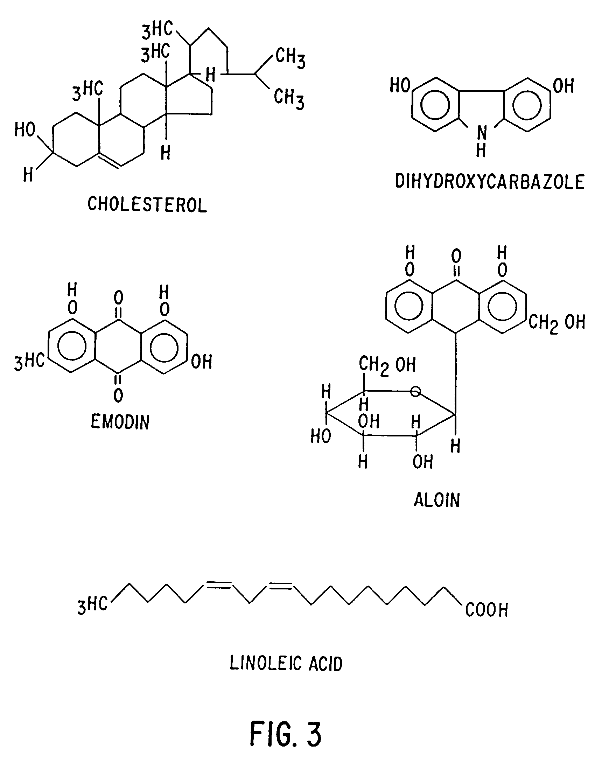

- Fig. 3:: Chemical structures of comonomers ("melanin-enhancing" agents) used in the present invention. Such agents can be copolymerized with one or more of the representative precursors in Fig. 1 to yield cosmetic melanins with enhanced penetration and substantivity to the skin over melanins produced without the "melanin-enhancing" agents. The melanin enhancing agents are drawn from different chemical classes (fatty acids, steroids, alkaloids, "aloins") but are generally both lipophilic (or possess lipophilic regions), hydroxylated, and/or exhibit unsaturated double bonds. Additional enhancing agents include extracts of Aloe Vera and related plants that are rich in aloins and emodins.

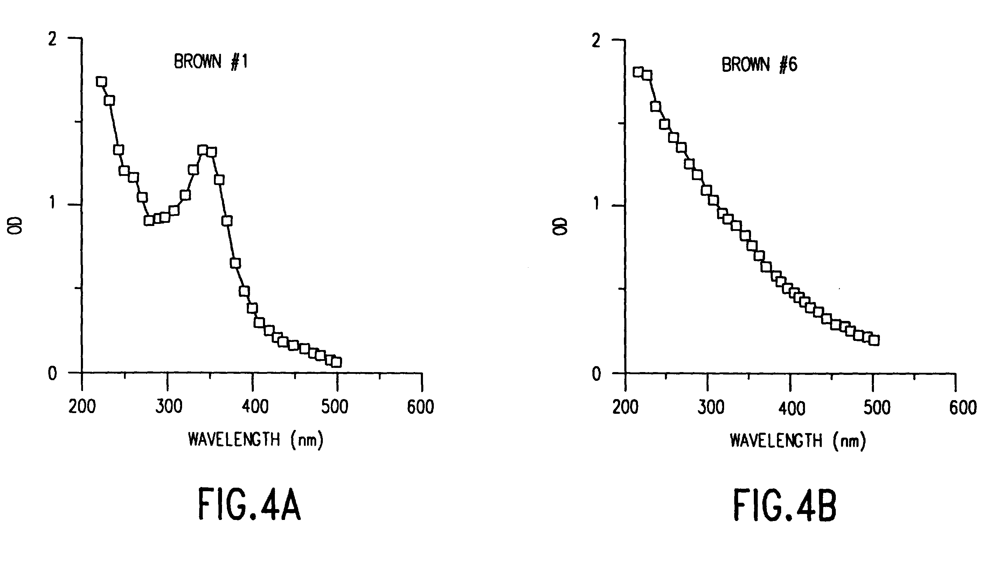

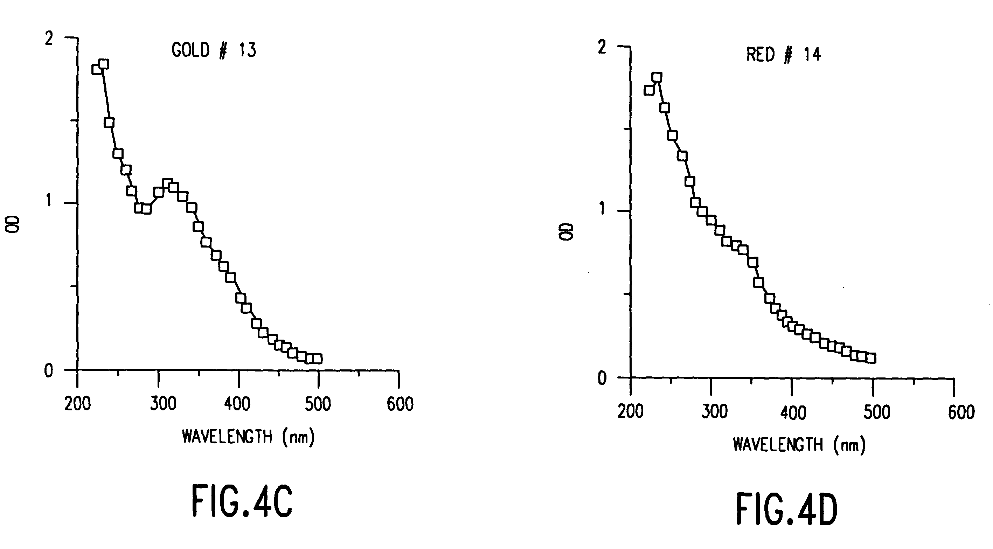

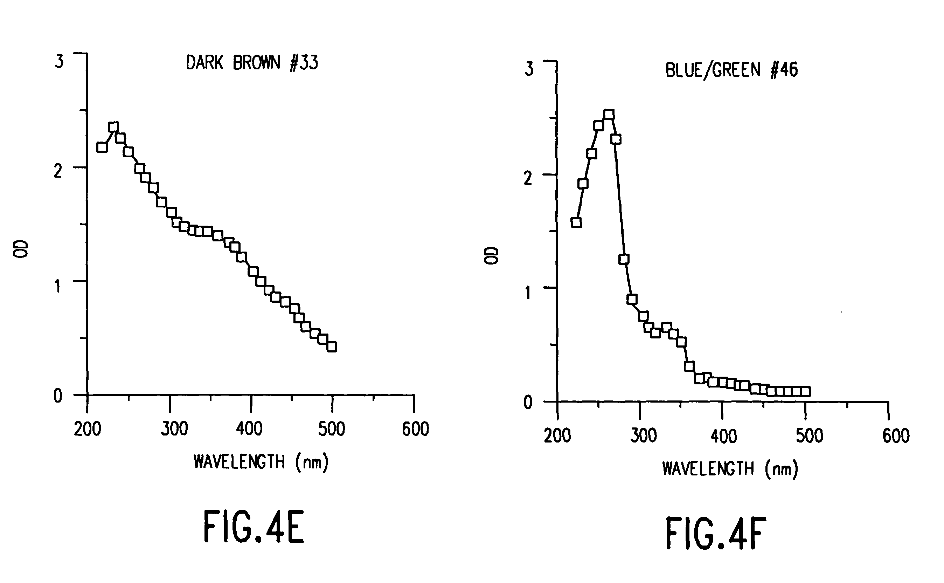

- Fig. 4:: Spectral characteristics of cosmetic melanins of various colors. Melanins were synthesized with or without "melanin-enhancing" agents by one of the six procedures listed above. Hence some are examples of the invention and some are not. They do all, however, illustrate a general finding relevant to the invention. Dried melanin powders were dissolved in 0.1M sodium phosphate, pH 7.4, at a concentration of 50µg/ml and absorbance was determined in a spectrophotometer over the range of wavelengths shown. Unlike the precursors shown in Fig. 1, each of the melanins exhibited absorbance in both the ultraviolet and visible spectra.

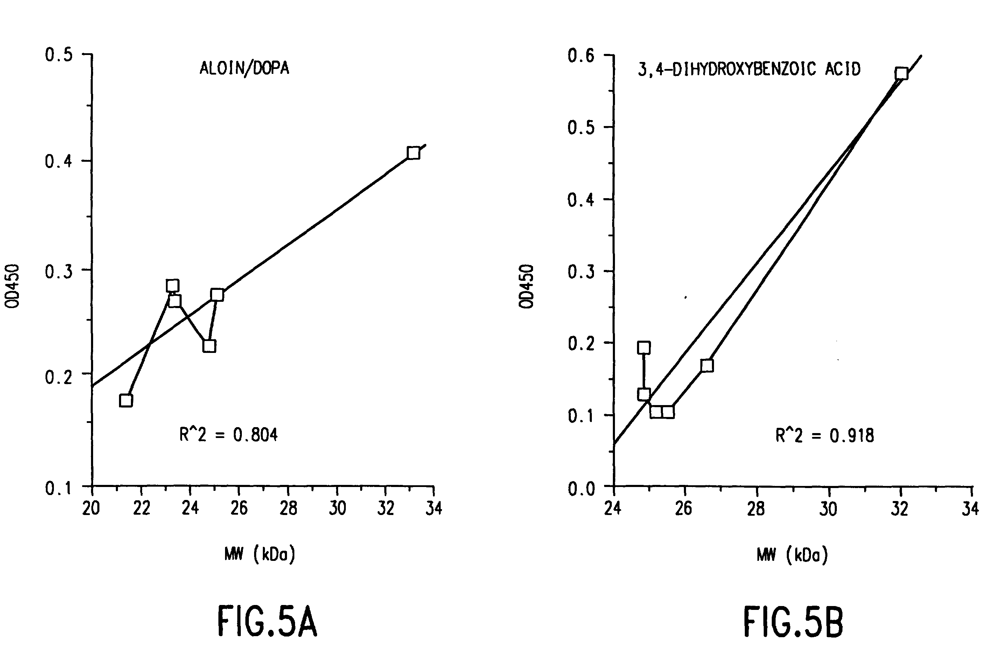

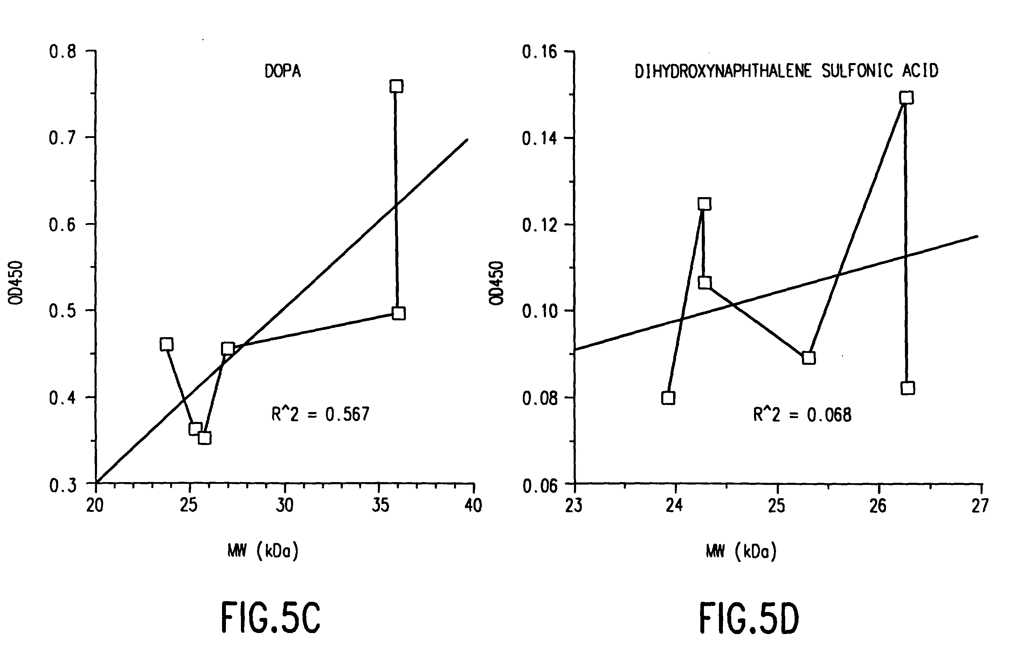

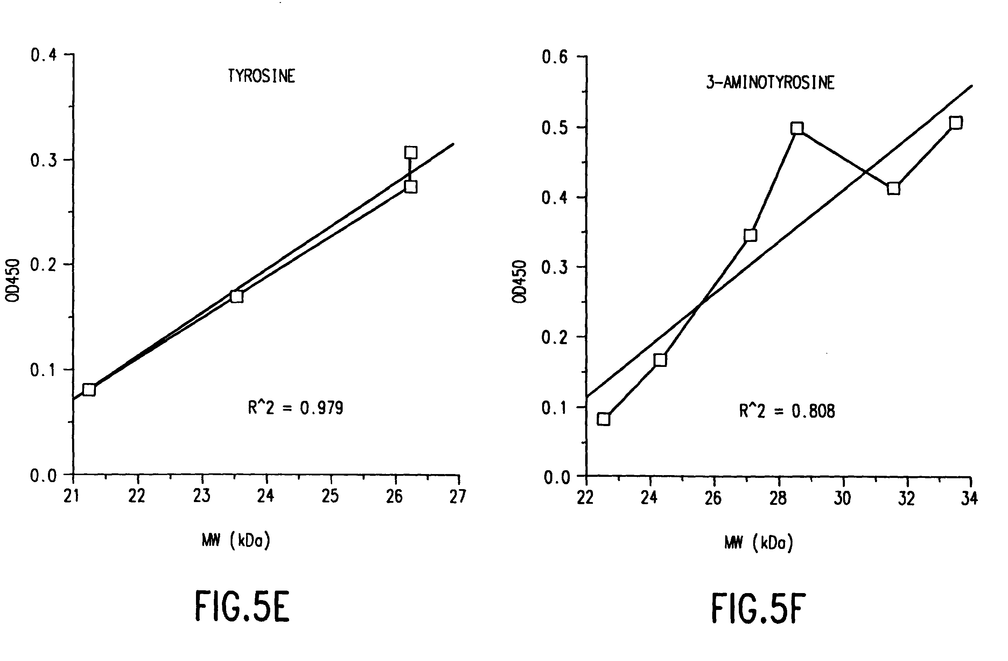

- Fig. 5:: Plots of molecular weights vs. absorbance (450nm) of melanin polymers. Only the polymer of Fig. 5A is a polymer in accordance with the present invention. Molecular weights were calculated via the elution times from a precalibrated molecular sieve HPLC column. Absorbance of a 50µg/ml sample was determined with a spectrophotometer. Generally, but not always, there was a correlation between the degree of absorbance and the degree of polymerization. Correlations were determined by the R² method of statistical analysis.

The molecular weights of each of the precursors and/or enhancing agents are all less than 1 kilodalton, whereas the molecular weights of the polymerized melanins average between 10-40 kilodaltons or higher. It can be seen from Table 1 that the same precursor can be polymerized into melanin polymers of different molecular weights, depending on which of the six polymerization procedures is used. Molecular weights of individual melanins were calculated via their elution times from a precalibrated molecular sieve column.

**Table 1**

| Molecular Weights (K daltons) of Melanin Polymers Synthesized Via Various Procedures | | | | | | |
|---|---|---|---|---|---|---|
| | Procedure: | | | | | |
| Precursor | 1 | 2 | 3 | 4 | 5 | 6 |
| A | NA | NA | 26.3 | 21.2 | 26.3 | 23.6 |
| B | 27.0 | 23.9 | 36.2 | 25.6 | 36.2 | 25.3 |
| C | 34.3 | 31.7 | ppt | ppt | 41.4 | 28.5 |
| D | 24.3 | 22.4 | 33.4 | 28.5 | 31.7 | 27.0 |
| E | 25.3 | 25.6 | 24.9 | 26.6 | 32.1 | 24.9 |
| F | 21.8 | 21.2 | 23.0 | 23.3 | 23.6 | 23.3 |
| G | 23.9 | 25.3 | 24.3 | 24.3 | 26.3 | 26.3 |
| H | NA | NA | 22.4 | NA | 23.6 | NA |
| I | 19.1 | 22.7 | 20.4 | 22.4 | 24.9 | 23.6 |
| J | 25.3 | 24.6 | 24.9 | 24.6 | 33.4 | 21.5 |

| | | | | | | |
|---|---|---|---|---|---|---|
| NA: negligible product | | | | | | |
| ppt: poorly soluble in aqueous buffer, pH 7.4 | | | | | | |
| (For the key for precursors A to J and Procedures 1 to 6 see Table 3.) | | | | | | |

The polymers formed from precursor J are the only polymers of the above Table which are in accordance with the invention. The others (precursors A to I) are not polymers of the present invention but do illustrate the general finding that procedures 1 to 6 to produce polymers of different molecular weights.

Likewise, the degree of absorbance in the visible spectrum per unit weight of melanin also differs according to the procedure and precursors employed in the polymerization reactions. In these runs, spectrophotometric absorbance at a wavelength of 450nm was used as a criterion of absorbance in the visible range. Absorbance of each individual melanin was measured by spectrophotometric absorbance of a solution of melanin dissolved at 50µg/ml of sodium phosphate, O.1M, pH 7.4. It can be seen that even with a single precursor, the absorbance of the resultant melanin polymers vary according to the procedure used.

The polymers formed from precursor J are the only polymers of the above Table which are in accordance with the invention. The others (precursors A to I) are not polymers of the present invention but do illustrate the general finding that procedures 1 to 6 produce polymers of different absorbances.

**Table 2**

| Spectrophotometric Absorbance (450nm) of Melanin Polymers (50µg/ml) Synthesized Via Various Procedures | | | | | | |
|---|---|---|---|---|---|---|
| | Procedure: | | | | | |
| Precursor | 1 | 2 | 3 | 4 | 5 | 6 |
| A | NA | NA | .275 | .084 | .308 | .170 |
| B | .456 | .461 | .763 | .350 | .496 | .358 |
| C | .450 | .293 | ppt | ppt | .457 | .457 |
| D | .167 | .074 | .507 | .500 | .411 | .343 |
| E | .101 | .100 | .194 | .171 | .577 | .128 |
| F | .145 | .291 | .733 | .757 | .930 | .445 |
| G | .078 | .089 | .125 | .106 | .150 | .082 |
| H | NA | NA | .493 | NA | .584 | NA |
| I | .158 | .210 | .312 | .280 | .326 | .221 |
| J | .261 | .223 | .302 | .373 | .217 | .191 |

| | | | | | | |
|---|---|---|---|---|---|---|
| NA: negligible product | | | | | | |
| ppt: poorly soluble in aqueous buffer, pH 7.4 | | | | | | |
| (For the key to precursors A to J and procedures 1 to 6 see Table 3.) | | | | | | |

Generally, but not always, there was a correlation between the degree of absorbance (450nm) and the degree of polymerization (molecular weight in kilodaltons). Examples of this are presented in Fig. 5 wherein the R² values are shown for plots of absorbance vs. molecular weights of various precursors. The closer the R² value is to 1.0, the higher the correlation value. In these examples, there is a correlation with each of the precursors except dihydroxynaphthalene sulfonic acid, whose absorbance characteristics show no correlation with the degree of polymerization. Therefore, the degree of polymerization is generally, but not always, proportional to the intensity of absorbance.

Using the screening procedures listed above, it was found that an array of colors can be produced, depending upon the precursor and the procedure used. Colors were determined from solutions of melanins dissolved at 5Oµg/ml sodium phosphate, O.1M, pH 7.4. The array of colors achieved represents a unique advance in melanin chemistry. Although each of the melanins absorbs light in both the ultraviolet and visible spectra, specific polymerization procedures can be used to bring out specific regions of the spectra, resulting in a variety of colors available for cosmetic uses. Discovery of these colors resulted from use of the six separate screening procedures set forth hereinabove.

Only polymers 100# and 55# shown in Table 3 are polymers in accordance with the present invention. The other polymers shown are not examples of the present invention. They do, however, illustrate the general finding that it is possible to obtain a range of colours by using different monomers and different procedures.

**Table 3**

| Colors of Melanins Obtained Via Various Synthetic Routes | | | | | | | |
|---|---|---|---|---|---|---|---|
| Melanin # | Precursor | Procedure # | Brown | Red | Gold | Olive | Blue/Green |
| 3 | A | 3 | + | | | | |
| 7 | B | 1 | + | | | | |
| 23 | C | 5 | + | | | | |
| 27 | D | 3 | + | | | | |
| 30 | D | 6 | + | | | | |
| 100 | E,F | 5 | + | | | | |
| 71 | I | 5 | + | | | | |
| 14 | E | 2 | | + | | | |
| 18 | E | 6 | | + | | | |
| 56 | E | 2 | | + | | | |
| 2 | A | 2 | | | + | | |
| 8 | B | 2 | | | + | | |
| 24 | C | 6 | | | + | | |
| 40 | G | 4 | | | + | | |
| 42 | G | 6 | | | ⁺ | | |
| 55 | E,F | 1 | | | + | | |
| 65 | H | 5 | | | + | | |
| 10 | B | 4 | | | | + | |
| 46 | G | 4 | | | | | + |
| Precursors: A) 3-aminotyrosine B) dihydroxyindole-2-carboxylic acid C) 3,4-dihydroxybenzoic acid D) 3-amino, 4-hydroxybenzoic acid E) Aloin F) dihydroxyphenylalanine G) 4,5-dihydroxynaphthalene-2-sulfonic acid H) 3-nitrotyrosine I) p-aminobenzoic acid J) aloin+DOPA Procedures: 1) weak base 2) weak base + salt of metal ion 3) weak base + salt of metal ion + oxidant 4) strong base 5) strong base + salt of metal ion 6) strong base + salt of metal ion + oxidant | | | | | | | |

Examples of spectral characteristics of melanins (not all Examples of the invention) exhibiting difference colors are shown in Fig. 4. It should be noted that even though specific colors exhibited by the different melanins (brown, red, gold, olive, blue/green), light absorbance in the ultraviolet spectra (approximately 200-340nm) was similar in each case.

The procedures for oxidative polymerization of suitable precursors into melanins or melanin-like compounds are readily amenable to industrial-scale production of the material. A straightforward and simple synthetic route comprises a) precursor (monomer) with an enhancer (comonomer); b) oxidative polymerization; c) concentration into powder; and d) formation into a suitable cosmetic vehicle.

The procedures involve, but are not limited to, mixing one or more precursors (e.g. see Fig. 1) with one or more enhancers (e.g. see Fig. 3), and subjecting the mixture to oxidative polymerization via one of the six procedures listed hereinabove. The resultant polymer is concentrated via precipitation by titration with an acid (e.g. HCl) or an alcohol (e.g. ethanol), by lyophilization or drying in air. The polymer is dried to a powder, and the powder is mixed at the desired concentration (e.g. 1% wt/wt) in a suitable cosmetic vehicle (e.g. Avon Body Lotion).

The polymers of the present invention so produced are believed to provide protection to the skin from damage caused by UV radiation (skin cancers, wrinkling, solar lentigines, skin aging) by virtue of the fact that they strongly absorb UV light. This general principle is illustrated by Figures 2 and 4 (see discussion above.) As mentioned above, PABA is used extensively by the industry as an active ingredient in sunscreen preparations, and it can be seen in Fig. 2 that melanins synthesized from PABA absorb UV light over a broader range than does PABA itself. In this regard, melanins prepared from precursors other than PABA have similar absorbance characteristics to PABA-melanin (of Figs. 2 and 4)

It can thus be concluded that since the polymers of the invention are both substantive to the skin and visible to the eye, they represent a significant and unique advantage over existing sunscreen formulations.

If desired, one part by weight of any of the polymers of the invention can be blended with about 1 to 10 and preferably about 1 to 5 and especially about 3 parts by weight of urea, the urea enhancing penetration of the melanin into the epidermis.

Any of the foregoing melanins can be mixed with one another to achieve a predetermined hue customized to a particular individual's skin and hair colors.

Accordingly, the present invention provides a method which comprises mixing a plurality of synthetic melanin polymers according to the invention to achieve a predetermined hue customized to a particular individual's skin and hair colours.

The novel melanins can be stored as dry powders and dissolved when ready for use, or can be stored as solutions. Advantageously, they are incorporated into liposomes in conventional manner and such liposomes used when required.

Accordingly the present invention provides a liposome containing a synthetic melanin polymer of the present invention.

The invention will be further described in the following illustrative examples wherein all parts are by weight unless otherwise expressed.

### Examples

### Example 1:

### Screening potential precursors to cosmetic melanins

In water, l-dopa (6.7% wt/vol) is mixed with aloin (3.3% wt/vol) and subjected to oxidative polymerization via each of six different procedures as follows: 1) in the presence of NH₄OH(5M); 2) in the presence of NH₄OH(5M) and CuSO₄ (0.6 mg/ml); 3) in the presence of NH₄OH(5M), CUS0₄ (0.6 mg/ml) and H₂0₂ (3% vol/vol); 4) in the presence of NaOH (1M); 5) in the presence of NaOH(1M) and CuSO₄ (0.6 mg/ml); 6) in the presence of NaOH(1M), CuSO₄ (0.6 mg/ml) and H₂0₂ (3%). The mixture is stirred vigorously in air for 24 hours. Two volumes of ethanol are added, the precipitate is collected by centrifugation, and lyophylized to dryness. The resultant powder is dissolved in Avon Body Lotion at a concentration of 5% (wt/wt; melanin/lotion) and applied to the skin and hair of a volunteer. Excess material is removed by washing with soap and water, and both the amount as well as the color of the adhering melanin is assessed visually.

### Example 2:

### Industrial production of a cosmetic melanin

Ingredients for the production of 100 kg (based on 100% yield) of a brown cosmetic melanin are listed as follows:
L-dihydroxyphenylalanine
67 kg
aloin
33 kg
NH₄0H (concentrated)
350 liters
CuSO₄
750 g
H₂O
550 liters
H₂0₂
100 liters (incrementally)

The ingredients are added in the order listed in a suitable mixing chamber. The mixture is aerated vigorously for 24 hours. The resultant melanin polymer is precipitated by the addition of ethanol (2.5 volumes, approximately 2500 liters). The precipitate is collected and dried in air. The melanin is then dissolved in Avon Body Lotion at a concentration of 1% (wt/wt; melanin/lotion), and packaged for use as a cosmetic melanin.

### Example 3:

### Application of cosmetic melanin to the skin and/or hair

a) A brown cosmetic melanin consisting of co-polymerized monomers of dopa (2 parts) and aloin (1 part) is mixed in Avon Body Lotion (1% wt/wt; melanin/lotion), and applied manually to the skin and/or hair of an individual desiring a natural-appearing brown or golden-brown coloration. Excess material is removed by washing with soap and water.
b) A red cosmetic melanin consisting of co-polymerized monomers of aloin (4 parts), 3-dimethylamino phenol (1 part) and linoleic acid (1 part) is mixed with Avon Body Lotion (5% wt/wt; melanin/lotion), and applied manually to the skin and/or hair of an individual desiring a natural-appearing red or golden-red coloration. Excess material is removed by washing with soap and water.
c) A blue/green cosmetic melanin consisting of monomers of dihydroxynaphthalenesulfonic acid (2 parts) and aloin (1 part) is mixed in Avon Body Lotion, and applied manually to the skin of an individual desiring this hue. A blue/green hue can be used to soften the appearance of blemishes. Excess material is removed by washing with soap and water.

### Example 4:

### Customized cosmetic melanins

Powdered forms of a brown melanin, a red melanin, and a blue/green melanin are separately dissolved in Avon Body Lotion at a concentration of 1% (wt/wt; melanin/lotion). The melanin-containing lotions are then mixed with one another in a variety of proportions (e.g. 1:1:1; 1:2:1; etc.) and applied to the skin and/or hair of a volunteer. When the desired hue is achieved, e.g. the color that most approximates that of the hair and eye color of the volunteer, the mixed proportions are recorded, and the melanin "blend" has thus been customized to the individual.

### Example 5:

### Use of cosmetic melanin as a protectant from ultraviolet radiation

A cosmetic melanin consisting of copolymerized monomers of dopa (3 parts), aloin (1 part), 3-dimethylamino phenol (1 part) and linoleic acid (1 part) is mixed with Avon Body Lotion (1% wt/wt; melanin/lotion) and applied to the skin of an individual. The inherent ultraviolet light absorbance of the melanin as well as its free radical scavenging characteristics provide protection from solar radiation to the individual's skin in the region wherein the melanin is applied.

## Claims

1. A synthetic melanin polymer comprising units derived from at least one monomer selected from 3-aminotyrosine, dihydroxybenzoic acid, 3-amino-4-hydroxybenzoic acid, tyrosine, dihydroxyphenylalanine, dihydroxynapththalene sulfonic acid, 3-nitrotyrosine, 3-dimethylamino phenol and p-aminobenzoic acid, and from at least one co-monomer selected from cholesterol, dihydroxycarbazole, aloin, emodin, linoleic acid and linolenic acid, the co-monomer(s) having been co-polymerized with the monomer(s), the polymer being substantive to human skin and hair and being soluble in an aqueous cosmetic buffer at physiological pH and temperature.

2. A synthetic melanin polymer according to claim 1, comprising units derived from dihydroxyphenylalanine and from aloin.

3. A synthetic melanin polymer according to claim 1, comprising units derived from tyrosine.

4. A synthetic melanin polymer according to claim 1, comprising units derived from dihydroxyphenylalanine.

5. A synthetic melanin polymer according to claim 1, comprising units derived from 3-amino-tyrosine.

6. A synthetic melanin polymer according to claim 1, comprising units derived from 3-nitro-tyrosine.

7. A synthetic melanin polymer according to claim 1, comprising units derived from dihydroxybenzoic acid.

8. A synthetic melanin polymer according to claim 1, comprising units derived from 3-amino-4-hydroxybenzoic acid.

9. A synthetic melanin polymer according to claim 1, comprising units derived from 3-dimethylamino phenol.

10. A synthetic melanin polymer according to claim 1, comprising units derived from p-aminobenzoic acid.

11. A synthetic melanin polymer according to claim 1, comprising units derived from dihydroxynaphthalene sulfonic acid.

12. A method of obtaining a synthetic melanin polymer, which comprises mixing at least one monomeric material selected from 3-aminotyrosine, dihydroxybenzoic acid, 3-amino-4-hydroxybenzoic acid, tyrosine, dihydroxyphenylalanine, dihydroxynaphthalene sulfonic acid, 3-nitrotyrosine, 3-dimethylamino phenol and p-aminobenzoic acid with at least one co-monomer selected from cholesterol, dihydroxycarbazole, aloin, emodin, linoleic acid and linolenic acid and polymerizing the mixture in aqueous solution by oxidation in the presence of air or oxygen:
(a) in the presence of a weak base; or
(b) in the presence of a weak base and a salt of Cu⁺⁺ or Fe⁺⁺; or
(c) in the presence of a weak base, a salt of Cu⁺⁺ or Fe⁺⁺ and an oxidant; or
(d) in the presence of a strong base; or
(e) in the presence of a strong base and a salt of Cu⁺⁺ or Fe⁺⁺; or
(f) in the presence of a strong base, a salt of Cu⁺⁺ or Fe⁺⁺ and an oxidant.

13. A method according to claim 12, wherein an alcohol is added to the resulting solution of formed polymer, thereby to precipitate the polymer from said solution.

14. A method according to claim 13, wherein the alcohol is ethanol.

15. A method which comprises mixing a plurality of synthetic melanin polymers according to any of claims 1 to 11 to achieve a predetermined hue customized to a particular individual's skin and hair colours.

16. A liposome containing a synthetic melanin polymer according to any one of claims 1 to 11.

17. A method of cosmetically producing a naturally appearing tan on mammalian skin comprising applying to the skin a tan-producing effective amount of a synthetic melanin polymer comprising units derived from at least one monomer selected from 3-aminotyrosine, dihydroxybenzoic acid, 3-amino-4-hydroxybenzoic acid, tyrosine, dihydroxyphenylalanine, dihydroxynapththalene sulfonic acid, 3-nitrotyrosine, 3-dimethylamino phenol and p-aminobenzoic acid, and from at least one co-monomer selected from cholesterol, dihydroxycarbazole, aloin, emodin, linoleic acid and linolenic acid, the co-monomer(s) having been co-polymerized with the monomer(s), the polymer being substantive to human skin and hair and being soluble in an aqueous cosmetic buffer at physiological pH and temperature.

## Patentansprüche

1. Ein synthetisches Melaninpolymer, das Einheiten aufweist, die sich von wenigstens einem Monomer, das ausgewählt ist aus 3-Aminotyrosin, Dihydroxybenzoesäure, 3-Amino-4-hydroxybenzoesäure, Tyrosin, Dihydroxyphenylalanin, Dihydroxynaphthalinsulfonsäure, 3-Nitrotyrosin, 3-Dimethylaminophenol und p-Aminobenzoesäure, sowie von wenigstens einem Co-monomer ableiten, das ausgewählt ist aus Cholesterin, Dihydroxycarbazol, Aloin, Emodin, Linolsäure und Linolensäure, wobei das oder die Co-monomer(e) mit dem oder den Monomer(en) copolymerisiert wurde(n), wobei das Polymer substantiv bezüglich der menschlichen Haut und des menschlichen Haars ist und bei physiologischem pH und physiologischer Temperatur in einem wäßrigen kosmetischen Puffer löslich ist.

2. Synthetisches Melaninpolymer nach Anspruch 1, das Einheiten aufweist, die sich von Dihydroxyphenylalanin und von Aloin ableiten.

3. Synthetisches Melaninpolymer nach Anspruch 1, das Einheiten aufweist, die sich von Tyrosin ableiten.

4. Synthetisches Melaninpolymer nach Anspruch 1, das Einheiten aufweist, die sich von Dihydroxyphenylalanin ableiten.

5. Synthetisches Melaninpolymer nach Anspruch 1, das Einheiten aufweist, die sich von 3-Aminotyrosin ableiten.

6. Synthetisches Melaninpolymer nach Anspruch 1, das Einheiten aufweist, die sich von 3-Nitrotyrosin ableiten.

7. Synthetisches Melaninpolymer nach Anspruch 1, das Einheiten aufweist, die sich von Dihydroxybenzoesäure ableiten.

8. Synthetisches Melaninpolymer nach Anspruch 1, das Einheiten aufweist, die sich von 3-Amino-4-hydroxybenzoesäure ableiten.

9. Synthetisches Melaninpolymer nach Anspruch 1, das Einheiten aufweist, die sich von 3-Dimethylaminophenol ableiten.

10. Synthetisches Melaninpolymer nach Anspruch 1, das Einheiten aufweist, die sich von p-Aminobenzoesäure ableiten.

11. Synthetisches Melaninpolymer nach Anspruch 1, das Einheiten aufweist, die sich von Dihydroxynaphthalinsulfonsäure ableiten.

12. Verfahren zur Gewinnung eines synthetischen Melaninpolymers, das das Vermischen von wenigstens einem Monomermaterial, das ausgewählt ist aus 3-Aminotyrosin, Dihydroxybenzoesäure, 3-Amino-4-hydroxybenzoesäure, Tyrosin, Dihydroxyphenylalanin, Dihyxdroxynaphthalinsulfonsäure, 3-Nitrotyrosin, 3-Dimethylaminophenol und p-Aminobenzoesäure, mit wenigstens einem Co-monomer, das ausgewählt ist aus Cholesterin, Dihydroxycarbazol, Aloin, Emodin, Linolsäure und Linolensäure, und die Polymerisation der Mischung in einer wäßrigen Lösung durch Oxidation in Gegenwart von Luft oder Sauerstoff umfaßt:
(a) in Gegenwart einer schwachen Base; oder
(b) in Gegenwart einer schwachen Base und eines Salzes von Cu⁺⁺ oder Fe⁺⁺; oder
(c) in Gegenwart einer schwachen Base, eines Salzes von Cu⁺⁺ oder Fe⁺⁺ und eines Oxidationsmittels; oder
(d) in Gegenwart einer starken Base; oder
(e) in Gegenwart einer starken Base und eines Salzes von Cu⁺⁺ oder Fe⁺⁺; oder
(f) in Gegenwart einer starken Base, eines Salzes von Cu⁺⁺ oder F⁺⁺ und eines Oxidationsmittels.

13. Verfahren nach Anspruch 12, bei dem der erhaltenen Lösung des gebildeten Polymers ein Alkohol zugesetzt wird, um dadurch das Polymer aus der Lösung auszufällen.

14. Verfahren nach Anspruch 13, bei dem der Alkohol Ethanol ist.

15. Verfahren, das das Vermischen einer Vielzahl von synthetischen Melaninpolymeren nach irgendeinem der Ansprüche 1 bis 11 umfaßt, um einen vorgegebenen Farbton zu erreichen, der den Haut- und Haarfarben eines speziellen Individuums individuell angepaßt ist.

16. Ein Liposom, das ein synthetisches Melaninpolymer nach irgendeinem der Ansprüche 1 bis 11 enthält.

17. Verfahren zur kosmetischen Erzeugung einer natürlich erscheinenden Bräunung auf einer Säugetierhaut, das das Aufbringen einer bräunungserzeugenden wirksamen Menge eines synthetischen Melaninpolymers auf die Haut umfaßt, das Einheiten aufweist, die sich von wenigstens einem Monomer, das ausgewählt ist aus 3-Aminotyrosin, Dihydroxybenzoesäure, 3-Amino-4 -hydroxybenzoesäure, Tyrosin, Dihydroxyphenylalanin, Dihydroxynaphthalinsulfonsäure, 3-Nitrotyrosin, 3-Dimethylaminophenol und p-Aminobenzoesäure, sowie von wenigstens einem Co-monomer ableiten, das ausgewählt ist aus Cholesterin, Dihydroxycarbazol, Aloin, Emodin, Linolsäure und Linolensäure, wobei das oder die Co-monomer(e) mit dem oder den Monomer(en) copolymerisiert wurde (n), wobei das Polymer bezüglich der menschlichen Haut und des menschlichen Haars substantiv ist und bei physiologischem pH und physiologischer Temperatur in einem wäßrigen kosmetischen Puffer löslich ist.

## Revendications

1. Polymère de mélamine synthétique comprenant des unités dérivées d'au moins un monomère sélectionné parmi la 3-aminotyrosine, l'acide dihydroxybenzoique, l'acide 3-amino-4-hydroxybenzoique, la tyrosine, la dihydroxyphénylalanine, l'acide dihydroxynaphtalène sulfonique, la 3-nitrotyrosine, le 3-diméthylamino phénol et l'acide p-aminobenzoique, et d'au moins un co-monomère sélectionné parmi le cholestérol, le dihydroxycarbazole, l'aloine, l'émodine, l'acide linoléique et l'acide linolénique, le(s) co-monomère(s) ayant été co-polymérisé(s) avec le(s) monomère(s), le polymère étant substantif à la peau humaine et aux cheveux et étant soluble dans un tampon cosmétique aqueux à pH et température physiologiques.

2. Polymère de mélamine synthétique selon la revendication 1, comprenant des unités dérivées de dihydroxyphénylalanine et d'aloine.

3. Polymère de mélamine synthétique selon la revendication 1, comprenant des unités dérivées de la tyrosine.

4. Polymère de mélamine synthétique selon la revendication 1, comprenant des unités dérivées de la dihydroxyphénylalanine.

5. Polymère de mélamine synthétique selon la revendication 1, comprenant des unités dérivées de la 3-amino-tyrosine.

6. Polymère de mélamine synthétique selon la revendication 1, comprenant des unités dérivées de la 3-nitro-tyrosine.

7. Polymère de mélamine synthétique selon la revendication 1, comprenant des unités dérivées de l'acide dihydroxybenzoique.

8. Polymère de mélamine synthétique selon la revendication 1, comprenant des unités dérivées de l'acide 3-amino-4-hydroxybenzoique.

9. Polymère de mélamine synthétique selon la revendication 1, comprenant des unités dérivées du 3-diméthylamino phénol.

10. Polymère de mélamine synthétique selon la revendication 1, comprenant des unités dérivées de l'acide p-aminobenzoique.

11. Polymère de mélamine synthétique selon la revendication 1, comprenant des unités dérivées de l'acide dihydroxynaphtalène sulfonique.

12. Méthode d'obtention d'un polymère de mélamine synthétique qui comprend le mélange d'au moins un matériau monomère sélectionné parmi la 3-aminotyrosine, l'acide dihydroxybenzoique. l'acide 3-amino-4-hydroxybenzoique, la tyrosine, la dihydroxyphénylalanine, l'acide dihydroxynaphtalène sulfonique, la 3-nitrotyrosine, le 3-diméthylamino phénol et l'acide p-aminobenzoique avec au moins un comonomère sélectionné parmi le cholestérol, le dihydroxycarbazole, l'aloine, l'émodine, l'acide linoléique et l'acide linolénique et la polymérisation du mélange en solution aqueuse par oxydation en présence d'air ou d'oxygène:
(a) en présence d'une base faible; ou
(b) en présence d'une base faible et d'un sel de Cu⁺⁺ ou de Fe⁺⁺; ou
(c) en présence d'une base faible, d'un de Cu⁺⁺ ou de Fe⁺⁺ et d'un oxydant; ou
(d) en présence d'une base forte; ou
(e) en présence d'une base forte et d'un sel de Cu⁺⁺ ou de Fe⁺⁺; ou
(f) en présence d'une base forte, d'un sel de Cu⁺⁺ ou de Fe⁺⁺ et d'un oxydant.

13. Méthode selon la revendication 12, où un alcool est ajouté à la solution résultante du polymère formé, pour ainsi précipiter le polymère de ladite solution.

14. Méthode selon la revendication 13, où l'alcool est l'éthanol.

15. Méthode qui comprend le mélange d'un certain nombre de polymères de mélanine synthétique selon l'une quelconque des revendications 1 à 11 pour obtenir une teinte prédéterminée produite spécialement selon les couleurs de peau et de cheveux d'un individu.

16. Liposome contenant un polymère de mélanine synthétique selon l'une quelconque des revendications 1 à 11.

17. Méthode de production cosmétique d'une couleur tan d'un aspect naturel sur une peau de mammalien comprenant l'application, à la peau, d'une quantité efficace de production de couleur tan d'un polymère de mélanine synthétique comprenant des unités dérivées d'au moins un monomère sélectionné parmi la 3-aminotyrosine, l'acide dihydroxybenzoique, l'acide 3-amino-4-hydroxybenzoique, la tyrosine, la dihydroxyphénylalanine. l'acide dihydroxynaphtalène sulfonique, la 3-nitrotyrosine, le 3-diméthylamino phénol et l'acide p-aminobenzoique, et d'au moins un comonomère sélectionné parmi le cholestérol, le dihydroxycarbazole, l'aloine, l'émodine, l'acide linoléique et l'acide linolénique, le(s) co-monomère(s) ayant été co-polymérisé(s) avec le(s) monomère(s), le polymère étant substantif pour la peau humaine et les cheveux et étant soluble dans un tampon cosmétique aqueux à pH et température physiologiques.
